# EUROPÄISCHE PATENTSCHRIFT

(11) **EP 2 306 948 B1**
(45) Veröffentlichungstag und Bekanntmachung des Hinweises auf die Patenterteilung: **06.12.2017**
(21) Anmeldenummer: 08773774.8
(22) Anmeldetag: 30.06.2008
(51) Int. Cl.: A61F 9/008, A61F 9/00

(54) **VORRICHTUNG ZUR OPHTHALMOLOGISCHEN, INSBESONDERE REFRAKTIVEN LASERCHIRURGIE**
DEVICE FOR OPHTHALMOLOGIC, PARTICULARLY REFRACTIVE, LASER SURGERY
DISPOSITIF DE CHIRURGIE LASER, NOTAMMENT RÉFRACTIVE, EN OPHTALMOLOGIE

(43) Veröffentlichungstag der Anmeldung: 13.04.2011
(73) Patentinhaber: WaveLight GmbH, 91058 Erlangen (DE)
(72) Erfinder: RIEDEL, Peter, 90489 Nürnberg (DE); DONITZKY, Christof, 90542 Eckental (DE)
(74) Vertreter: Katérle, Axel
(86) Internationale Anmeldenummer: PCT/EP2008/005333
(87) Internationale Veröffentlichungsnummer: WO 2010/000279

(56) Entgegenhaltungen:
- WO-A-01/19303
- WO-A-99/65431
- WO-A-2008/087483
- WO-A1-2007/016231
- WO-A1-2008/109627
- US-A1- 2005 024 586
- US-A1- 2005 140 981
- US-A1- 2006 100 613
- US-A1- 2006 195 076

## Beschreibung

Die Erfindung betrifft eine Vorrichtung für die Laserablation einer humanen Kornea.

Die Chirurgie des humanen Auges kennt zahlreiche Behandlungsmethoden, bei denen Laserstrahlung auf das Auge gerichtet wird, um infolge der Wechselwirkung der eingestrahlten Laserstrahlung mit dem Auge einen indizierten Behandlungszweck zu erreichen. Bei der refraktiven Laserchirurgie ist der Behandlungszweck eine Änderung der Abbildungseigenschaften des optischen Systems "Auge" mittels der Laserstrahlung. Da für die Abbildungseigenschaften des humanen Auges vor allem die Hornhaut (Kornea) maßgeblich ist, umfasst die refraktive Laserchirurgie des Auges in vielen Fällen eine Bearbeitung der Kornea. Durch gezieltes Einbringen von Schnitten und/oder durch gezielten Materialabtrag wird dabei eine Formänderung der Kornea bewirkt; man spricht deshalb auch von einer Neuformung.

Ein bekanntes Beispiel einer Neuformung der Kornea zur Änderung ihrer refraktiven Eigenschaften ist die Lasik (Laser in-situ Keratomileusis). Bei der Lasik wird aus der Kornea ein oberflächliches Deckelscheibchen herausgeschnitten, das in der Fachwelt gemeinhin als Flap bezeichnet wird. Der Flap hängt an einem Teil seines Rands in einem Scharnierbereich (englisch: hinge) noch an dem daneben liegenden Hornhautgewebe, sodass er problemlos zur Seite geklappt und später wieder zurückgeklappt werden kann. Zur Erzeugung des Flaps kommen in der bisherigen Praxis insbesondere zwei Methoden zum Einsatz, zum einen eine mechanische mittels eines Mikrokeratoms und zum anderen eine lasertechnische, bei der mittels Femtosekunden-Laserstrahlung (d.h. gepulster Laserstrahlung mit einer Pulsdauer im fs-Bereich) ein flächiger Tiefenschnitt in die Kornea eingebracht wird, der ausgenommen des Scharnierbereichs zur Korneaoberfläche herausgeführt wird. Nach Wegklappen des erzeugten Flaps erfolgt ein Materialabtrag (Ablation) vom so freigelegten Stroma gemäß einem vorher für den Patienten festgelegten Ablationsprofil. Das Ablationsprofil gibt an, an welcher Stelle der Kornea wie viel Gewebe abzutragen ist. Es wird so berechnet, dass nach der Ablation die Kornea eine für das behandelte Auge optimale Form hat und die zuvor vorhandenen optischen Abbildungsfehler des Auges weitestmöglich korrigiert sind. Für die Ablation kommt beispielsweise ein Excimer-Laser mit einer Strahlungswellenlänge im UV-Bereich zum Einsatz, etwa bei 193 nm.

Für die Durchführung der Ablation - oder allgemein gesagt, für die Durchführung einer Laserbehandlung des Auges - muss der Patient zunächst geeignet positioniert werden, so dass sein Auge einen bestimmten Arbeitsabstand zu der Laservorrichtung hat. Eine photodisruptive oder ablatierende Wechselwirkung der Laserstrahlung mit dem behandelten Gewebe tritt allein im Bereich des Strahlfokus auf. Der Arbeitsabstand ist deshalb so zu wählen, dass die zu behandelnde Stelle des Auges im wesentlichen in der Ebene des Strahlfokus liegt (als Ebene des Strahlfokus ist hier diejenige Ebene gemeint, die senkrecht zur Strahlrichtung liegt und durch den Strahlfokus hindurchgeht). Wenn der Kopf des Patienten nicht korrekt relativ zur Ebene des Strahlfokus positioniert ist, vergrößert sich der Strahldurchmesser an der Stelle des Auges, wo die gewünschte Behandlung stattfinden soll. Die Bestrahlungsstärke nimmt dementsprechend ab, d.h. es wird weniger Energie pro Fläche eingestrahlt. Dies kann zu einer unzureichenden Ablation und zu Unregelmäßigkeiten im Abtrag führen. Letztlich ergibt sich ein verschlechtertes Behandlungsergebnis.

Zur Ausrichtung des Patienten gegenüber der Laservorrichtung ist eine Vorgehensweise bekannt geworden, bei der zwei sich kreuzende Hilfs-Lichtstrahlen von seitlich oberhalb auf das behandelnde Auge gerichtet werden. Die Quellen dieser Hilfs-Lichtstrahlen, z.B. schwache Laserdioden, sind so angeordnet, dass sich die kornealen Reflexe der Hilfs-Lichtstrahlen in einem gemeinsamen Punkt auf der Hornhautoberfläche vereinen, vorausgesetzt, das Auge ist präzise in dem gewünschten Arbeitsabstand von der Laservorrichtung positioniert. Bei einer Fehlpositionierung des Patienten sind dagegen zwei Einzelreflexe auf der Kornea sichtbar, je einer von jedem Hilfs-Lichtstrahl. Dies gilt sowohl dann, wenn sich der Patient zu nah an der Laservorrichtung befindet, als auch dann, wenn er sich zu weit weg befindet. Einmal liegt der Kreuzungspunkt der Hilfs-Lichtstrahlen vor der Kornea, einmal dahinter. Dies zeigt, dass mit den beiden Hilfs-Lichtstrahlen zwar die richtige Positionierung des Patienten erkennbar ist, bei einer Fehlpositionierung aber nicht ohne weiteres erkennbar ist, in welche Richtung die Abweichung geht. Dies kann nur herausgefunden werden, indem der Patient testweise verfahren wird. Findet in der einen Verfahrrichtung keine Annäherung der Reflexe statt, muss er in der anderen Richtung verfahren werden. Es ist überdies nicht ohne weiteres erkennbar, wie stark die Abweichung des Patienten von der optimalen Arbeitslage ist. Der gegenseitige Abstand der Reflexe auf der Hornhaut kann zwar unter Umständen ein grober Anhaltspunkt sein, er erlaubt jedoch keinerlei quantitative Aussagen.

Die obigen Schwierigkeiten für den behandelnden Arzt, Richtung und Ausmaß einer Fehlpositionierung des Patienten präzise aus dem Bild der Reflexe auf der Kornea zu beurteilen, führen letztlich zu zahlreichen Justagebewegungen, die an der Patientenliege vorgenommen werden müssen, um den optimalen Arbeitsabstand für den Patienten aufzufinden.

Dokument US 2005/024586 A1 betrifft ophthalmologische Diagnoseverfahren oder - vorrichtungen, die eine Hochgeschwindigkeits-Augentracking-Vorrichtung zum Messen schneller Bewegungen des Auges und eine Augenpositionsmessvorrichtung kombinieren, wobei die Augenpositionsmessvorrichtung mehrere Dimensionen einer Augenposition oder anderer Komponenten des Auges relativ zu einem ophtalmologischen Diagnostik oder Behandlungsvorrichtung bestimmt.

Dokument WO 01/19303 A betrifft ein Verfahren zur Fotoablation der Kornea mit einem Laserstahl, mit dem eine Vielzahl hintereinander folgender Teilablationsvorgänge vorgenommen werden, sowie eine Vorrichtung mit einer Laserstrahlungsquelle zur Durchführung dieses Verfahrens. Mit der Erfindung können einmal intraokulare Dicken und Distanzen im vorderen Augenabschnitt unmittelbar vor, während und unmittelbar nach chirurgischen Eingriffen und Behandlungen der Kornea gemessen werden. Abhängig von diesen Messergebnissen kann eine Steuerung einer photorefraktiven Behandlung in Echtzeit erfolgen. Die Steuerung in Echtzeit führt zu einer erhöhten Sicherheit für den Patienten und einer verbesserten Genauigkeit der photorefraktiven Augenkorrektur.

Dokument WO 2008/109627 A1 betrifft ein System zum Positionieren eines Auges eines Patienten für Laserophtalmologische Operationen, umfassend ein Reflektometer zum Erhalten eines reflektierten Strahls von einer vorderen Oberfläche einer Kornea eines Auges eines Patienten als Eingangssignal. Das Interferometer ist auf eine Soll-Position der äußeren Oberfläche der Kornea kalibrierbar. Ein Vergleicher steht in Kommunikation mit dem Interferometer und ist ausgebildet zum Berechnen einer Differenz aus dem Eingangssignal einer aktuellen Position und der Soll-Position der vorderen Oberfläche der Kornea. Eine Vorrichtung steht in Kommunikation mit dem Vergleicher zum Bewegen des Patienten um eine Distanz in eine Richtung zum Übereinbringen der aktuellen Position mit der Soll-Position der vorderen Oberfläche der Kornea.

Dokument WO 2007/016231 A1 betrifft ein System und Verfahren zum Bestimmen einer optimalen Position eines Auges relativ zu einer ophtalmologischen Vorrichtung. Ein Aspekt des Verfahrens beinhaltet das Empfangen von Daten, die ein Bild der Oberfläche eines Auges bei einer ersten Position des Auges relativ zu einer ophtalmologischen Vorrichtung umfassen. Ein Merkmal in dem Bild wird lokalisiert, und eine Schärfekorrektur des Merkmals wird ausgeführt, indem ein vorbestimmter Algorithmus zum Erhalten eines Schärfewerts angewandt wird. Die Augenoberfläche wird dann auf eine zweite Position relativ zur ophtalmologischen Vorrichtung justiert, und die vorangegangen Schritte werden so lange wiederholt, bis der Schärfewert ein Maximum erreicht, was ein Hinweis auf eine erreichte optimale Augenposition ist.

Aufgabe der Erfindung ist es, die präzise Positionierung des Patienten für die ophthalmologische Laserchirurgie zu vereinfachen.

Zur Lösung dieser Aufgabe sind erfindungsgemäß Vorrichtungen gemäß den Ansprüchen 1 und 2 vorgesehen. Die Vorrichtungen umfassen jeweils:
- eine im UV-Wellenlängenbereich strahlende Excimer-Laserstrahlquelle zur Abgabe eines fokussierten Behandlungslaserstrahls,
- eine kohärenzoptische interferometrische Messeinrichtung zum Messen des Abstands eines vorgegebenen Messpunkts oder Messbereichs eines zu behandelnden Auges von einem in bekanntem Zusammenhang zur Fokuslage des Behandlungslaserstrahls stehenden Referenzpunkt, und
- eine Auswerte- und Steuereinheit, welche dazu eingerichtet ist, auf Grundlage der Abstandsmessdaten der Messeinrichtung das Zusammenfallen oder Auseinanderfallen eines gewünschten Einwirkorts des Behandlungslaserstrahls am oder im Auge und der Fokuslage des Behandlungslaserstrahls zu erfassen und im Fall des Zusammenfallens oder/und des Auseinanderfallens eine vorbestimmte Ausgabeaktion zu bewirken.

Kohärenzoptische interferometrische Messeinrichtungen stehen seit einiger Zeit zur berührungslosen Vermessung von Augenparametern, z.B. der Hornhautdicke oder der Vorderkammertiefe, zur Verfügung. Solche Messeinrichtungen arbeiten beispielsweise nach dem Prinzip der optischen Kurzkohärenzreflektometrie (OLCR: Optical Low-Coherence Reflectometry) oder der optischen Kohärenztomographie (OCT: Optical Coherence Tomography). Sie arbeiten mit kurzkohärenter, breitbandiger Messstrahlung und gestatten es, Strukturen des Auges (oder allgemein des zu vermessenden biologischen Gewebes) mit einer Auflösung im Bereich von 1 µm und feiner zu vermessen. Die OLCR eignet sich dabei insbesondere auch für Abstandsmessungen zwischen dem Auge und der laserchirurgischen Vorrichtung. Deshalb basiert bei der Erfindung die Messeinrichtung vorzugsweise auf OLCR.

Die Erfindung lehrt die Integration einer Messeinrichtung der obigen Art in eine laserchirurgische Vorrichtung und die Nutzung der Messeinrichtung zur Ausrichtung des Patienten. Die Messdaten einer OLCR- oder anderen interferometrischen Messeinrichtung gestatten die Bestimmung von Richtung und Stärke einer Abweichung des tatsächlichen Patientenabstands vom optimalen Arbeitsabstand und machen somit die Justierung des Patienten einfacher. Die Messeinrichtung misst zweckmäßigerweise den Abstand des Auges von dem Referenzpunkt in Richtung des Behandlungslaserstrahls (z-Richtung). Der Referenzpunkt ist ein gegebener, in dem Koordinatensystem der laserchirurgischen Vorrichtung eindeutig lokalisierbarer Punkt, z. B. der Koordinatenursprung oder ein vom Ursprung verschiedener Punkt. Der Strahlfokus des Behandlungslaserstrahls wird ebenfalls mit Bezug auf das Koordinatensystem der laserchirurgischen Vorrichtung eingestellt. Die Auswerte- und Steuereinheit kann so aus dem gemessenen Augenabstand und der z-Lage des Strahlfokus den z-Abstand des Auges von der Fokusebene ermitteln. Insbesondere kann die Auswerte- und Steuereinheit ermitteln, ob der gewünschte Einwirkort des Behandlungslaserstrahls in die Fokusebene fällt oder gegenüber dieser versetzt ist.

Der Punkt oder Bereich des Auges, von welchem aus die Messeinrichtung den Abstand zu dem Referenzpunkt misst, liegt z.B. auf der Hornhautoberfläche des Auges. Soll die Laserbehandlung oberflächennah stattfinden, wie dies etwa bei der Ablation im Rahmen der Lasik der Fall ist, so kann der gewünschte Einwirkort, auf den die Fokus- bzw. Behandlungsebene des Behandlungslaserstrahls einzustellen ist, als im Wesentlichen gleich angesehen werden wie der Messpunkt des Auges, dessen Abstand von dem Referenzpunkt gemessen wird. Es kann auch sein, dass ein unterhalb der Hornhautoberfläche liegender Punkt als Zielpunkt für die Abstandsmessung herangezogen wird, beispielsweise bei der Femtosekunden-LASIK.

Die Erfindung ist jedoch keineswegs auf eine laserchirurgische Vorrichtung für die Lasik beschränkt. Sie ist genauso anwendbar bei laserchirurgischen Vorrichtungen für andere ophthalmologische Behandlungszwecke. Es kann demnach sein, dass der gewünschte Einwirkort des Behandlungslaserstrahls ein anderer Ort des Auges ist als derjenige Punkt, von dem aus der Abstand zu dem Referenzpunkt gemessen wird. Wie gesagt, für die Abstandsmessung eignet sich beispielsweise ein Punkt an der Hornhautoberfläche, etwa der Apex (Scheitelpunkt) der Kornea. Der gewünschte Einwirkort des Behandlungslaserstrahls kann dagegen tiefer im Auge liegen, etwa an der Linse. In diesem Fall ist es zweckmäßig, den z-Abstand zwischen dem Messpunkt des Auges und dem gewünschten Einwirkort bei der Patientenausrichtung zu berücksichtigen. Unter der Voraussetzung, dass dieser z-Abstand bekannt ist, etwa durch eine vorhergehende OLCR-Vermessung des Auges, kann die Auswerte- und Steuereinheit aus dem gemessenen Augenabstand von dem Referenzpunkt, der bekannten relativen Lage des Strahlfokus gegenüber dem Referenzpunkt und dem ebenfalls bekannten z-Abstand zwischen dem Augenmesspunkt und dem gewünschten Einwirkort ohne Weiteres feststellen, ob die Fokusebene mit dem gewünschten Einwirkort in z-Richtung zusammenfällt oder nicht.

Verschiedene Ausgabeaktionen der Auswerte- und Steuereinheit sind denkbar, wenn ein Zusammenfallen des gewünschten Einwirkorts mit dem Fokus in z-Richtung festgestellt wird oder ein Auseinanderfallen. Für eine Dokumentation und Protokollierung der relativen Lage der Fokusebene (Behandlungsebene) zum gewünschten Einwirkort des Behandlungslaserstrahls kann eine Form von Ausgabeaktion der Auswerte- und Steuereinheit in der Ausgabe von Speicher- oder Anzeigebefehlen bestehen, die eine Speicherung bzw. Anzeige der Messergebnisse auf einer Anzeigeeinheit bewirken. Eine solche Dokumentation ist insbesondere dann zweckmäßig, wenn während der Laserbehandlung mehrere Abstandsmessungen durchgeführt werden, beispielsweise in regelmäßigen zeitlichen Intervallen. Denn es kann nicht ausgeschlossen werden, dass der Patient während der Laserchirurgie seinen Kopf bewegt und so eine Fehlausrichtung herbeiführt, die dem Operateur nicht verborgen bleiben sollte. Die gespeicherten Messergebnisse können bei Bedarf im Anschluss an die Operation ausgedruckt oder anderweitig verwendet werden. Eine sofortige Anzeige des jeweils aktuell gemessenen Abstands auf einer Anzeigeeinheit gibt dem Operateur eine Prüfmöglichkeit an die Hand, die es ihm gestattet, bei Bedarf korrigierend in den Behandlungsablauf einzugreifen oder diesen unter Umständen sogar zu unterbrechen.

Bei einer Ausführungsform der Erfindung ist eine Automatisierung dahingehend vorgesehen, dass die Auswerte- und Steuereinheit in Antwort auf das Ergebnis der Abstandsmessung einen Steuerbefehl an die Laserstrahlquelle ausgeben kann, um die Abgabe des Behandlungslaserstrahls zu steuern. Beispielsweise kann ein solcher Steuerbefehl die Abgabe des Behandlungslaserstrahls nur unter der Bedingung gestatten, dass der gewünschte Einwirkort und die Fokuslage im Wesentlichen zusammenfallen. Dies ist zweckmäßig, um zu gewährleisten, dass die Laserchirurgie erst dann begonnen wird, wenn der Patient korrekt ausgerichtet ist. Alternativ oder zusätzlich kann die Auswerte- und Steuereinheit dazu eingerichtet sein, Steuerbefehle abzugeben, welche eine Unterbrechung der Abgabe des Behandlungslaserstrahls bewirken, falls die Abstandsmessung ergibt, dass der gewünschte Einwirkort und die Fokuslage auseinanderfallen. Die Auswerte- und Steuereinheit kann so die Laserchirurgie automatisch unterbrechen, falls der Patient während der Behandlung seinen Kopf bewegt.

Selbstverständlich sind verschiedene andere Arten von Ausgabeaktionen der Auswerte- und Steuereinheit vorstellbar. Beispielsweise kann die Auswerte- und Steuereinheit dazu eingerichtet sein, die Ausgabe einer optischen oder/und akustischen Signalmeldung zu bewirken, wenn im Rahmen der Abstandsmessung ein Zusammenfallen oder ein Auseinanderfallen des gewünschten Einwirkorts und der Fokuslage festgestellt wird.

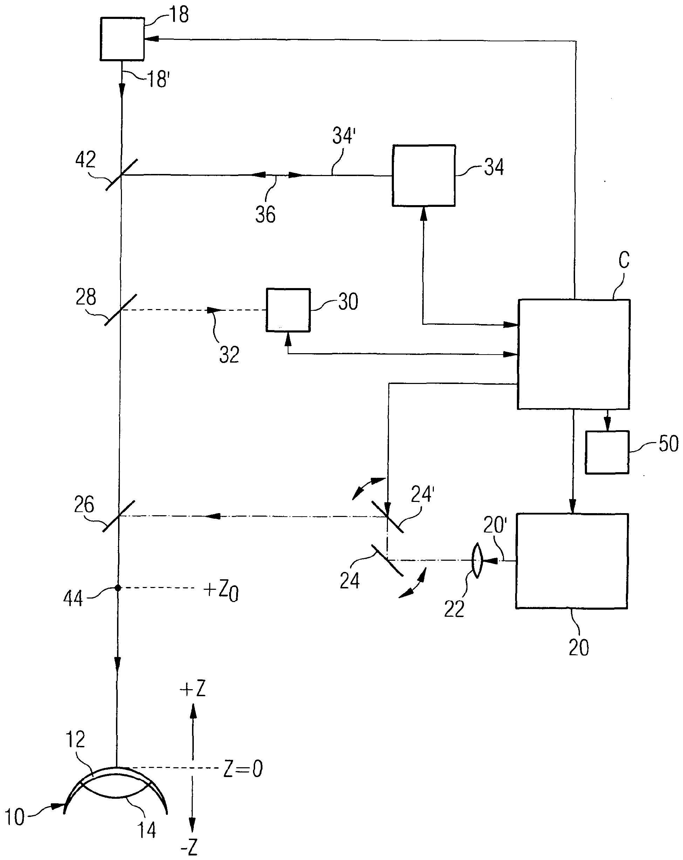

Die Erfindung wird im Folgenden anhand der beigefügten einzigen Zeichnung weiter erläutert. Diese zeigt schematisch ein Ausführungsbeispiel einer erfindungsgemäßen laserchirurgischen Vorrichtung, die sich insbesondere, jedoch nicht ausschließlich, für die Durchführung der Ablation bei der Lasik eignet.

Dargestellt ist in der Zeichnung zunächst ein humanes Auge 10 mit einer Kornea 12 und einem Pupillenrand 14.

Die gezeigte laserchirurgische Vorrichtung weist in an sich bekannter Weise eine Fixationslichtquelle (z.B. LED, Laser) 18 auf, die einen (schwachen) Fixationslichtstrahl 18' emittiert und vom Patienten zur Fixierung des Auges anvisiert wird.

Ferner umfasst die laserchirurgische Vorrichtung einen Behandlungslaser 20, der einen Behandlungslaserstrahl 20' emittiert, welcher über eine Linse 22 auf ein Paar Scanner-Spiegel 24, 24' gerichtet und über einen teildurchlässigen Umlenkspiegel 26 auf das Auge 10 gelenkt wird. Der Behandlungslaserstrahl 20' ist ein fokussierter Laserstrahl; eine Fokussieroptik zur Fokussierung des Behandlungslaserstrahls 20' ist in der Zeichnung nicht näher dargestellt. Geeignete Fokussieroptiken sind jedoch als solche im Stand der Technik hinlänglich bekannt.

Der Laser 20 ist ein Excimer-Laser, dessen Strahlungswellenlänge im UV-Bereich liegt, etwa bei 193 nm.

Die Scanner-Spiegel 24, 24' sind beispielsweise galvanometrisch betätigt und werden zusammen mit dem Laser 20 von einem programmgesteuerten Rechner C gemäß einem zuvor berechneten Behandlungsprofil (d.h. einem Ablationsprofil) gesteuert. Der Rechner C stellt eine Auswerte- und Steuereinheit im Sinne der Erfindung dar.

Die laserchirurgische Vorrichtung besitzt darüber hinaus eine Einrichtung zur Verfolgung von Augenbewegungen (Eye-Tracker). Der Eye-Tracker umfasst eine Kamera 30, mit der über einen teildurchlässigen Umlenkspiegel 28 in Richtung eines Pfeils 32 Bilder des Auges, konkret der Pupille und der Iris, aufgenommen werden können. Die Bilddaten der Kamera 30 werden sodann in dem Rechner C mittels Bildanalysesoftware ausgewertet, um Bewegungen des Auges, die der Patient trotz der versuchten Blickfixierung auf das Fixationslicht 18' in der Regel nicht vermeiden kann, zu verfolgen. Die detektierten Augenbewegungen berücksichtigt der Rechner C bei der Steuerung der Scanner-Spiegel 24, 24', um so das Bearbeitungsprofil möglichst konstant gegenüber einem beispielsweise an der Korneaoberfläche liegenden vorgegebenen Referenzpunkt des Auges ausgerichtet zu halten.

In die laserchirurgische Vorrichtung ist zudem eine Pachymeter-Messeinrichtung 34 für die optische Kurzkohärenzreflektormetrie (OLCR: Optical Low Coherence Reflectometry) integriert, die in an sich bekannter Weise eine Strahlungsquelle (z.B. SLED, ASE, Supercontinuum-Laser) enthält, der einen Messstrahl 34' abgibt. Der Messstrahl wird über einen teildurchlässigen Umlenkspiegel 42 auf das Auge 10 gerichtet, nämlich so, dass er gleichachsig zum Strahlengang des Behandlungslaserstrahls 20' auf das Auge fällt. Die Messeinrichtung 34 empfängt vom Auge 10 reflektierte Messstrahlung über den Umlenkspiegel 42 auf dem gleichen Weg, auf dem der Messstrahl 34' von der Messeinrichtung 34 ausgesendet wird. Dies ist durch einen Doppelpfeil 36 veranschaulicht.

Die Messeinrichtung 34 misst im Rahmen einer mittels der laserchirurgischen Vorrichtung durchzuführenden Behandlung unter Steuerung des Rechners C mindestens einmal, vorzugsweise mehrere Male den Abstand des Auges 10 in Richtung der Strahlachse des auf das Auge treffenden Teils des Behandlungslaserstrahls 20' (z-Richtung) von einem vorgegebenen Referenzpunkt im Koordinatensystem der laserchirurgischen Vorrichtung. Zum Zwecke der Veranschaulichung ist der Referenzpunkt in der Zeichnung bei 44 angedeutet. Er befindet sich an einer z-Position +z₀, deren Wert bekannt ist. Als Messpunkt am Auge 10 dient vorzugsweise ein Punkt an der Hornhautoberfläche, beispielsweise der Apex.

Die Messeinrichtung 34 liefert ihre Abstandsmessdaten an den Rechner C, der den gemessenen z-Abstand zwischen dem Augenmesspunkt und dem koordinatensystemfesten Referenzpunkt 44 mit dem z-Abstand zwischen dem Strahlfokus des Laserbehandlungsstrahls 20' und dem Referenzpunkt 44 vergleicht. Die z-Position des Strahlfokus ist durch die Einstellung der erwähnten Fokussieroptik vorgegeben und dem Rechner C bekannt. Im vorliegenden Fall sei beispielhaft angenommen, dass der Strahlfokus auf eine Position bei z=0 eingestellt ist. Ergibt der Abstandsvergleich, dass der zu behandelnde Teil des Auges 10 in der Fokusebene des Behandlungslaserstrahls 20' liegt, gibt der Rechner C beispielsweise den Laser 20 frei, so dass die Laserchirurgie beginnen kann. Ergibt der Abstandsvergleich jedoch, dass das Auge 10 nicht korrekt in z-Richtung positioniert ist, d.h. der zu behandelnde Teil des Auges nicht in der Fokusebene liegt, so bewirkt der Rechner C beispielsweise eine Anzeige des Justierbedarfs auf einer Anzeigeeinheit 50. Der angezeigte Justierbedarf gibt dem Operateur Richtung und Maß der nötigen z-Verstellung des Patienten an. Der Operateur kann dann durch vertikale Verstellung einer Patientenliege, auf welcher der Patient liegt, die optimale Patientenposition anfahren. Dabei ist es zweckmäßig, wenn die Messeinrichtung 34 fortlaufend weitere Abstandsmessungen durchführt und der Rechner C die jeweils aktuelle z-Lage des Auges 10 relativ zur Fokusebene auf der Anzeigeeinheit 50 anzeigt, um dem Operateur eine sofortige Kontrolle des Erfolgs seiner Justierbemühungen so ermöglichen. Sobald der Patient so positioniert ist, dass die gewünschte Behandlungsstelle des Auges und der Strahlfokus in z-Richtung im Wesentlichen zusammenfallen, kann dies dem Operateur beispielsweise durch einen gut hörbaren Signalton zusätzlich mitgeteilt werden.

Es ist selbstverständlich auch möglich, den Verstellmechanismus der Patientenliege mit dem Rechner C zu koppeln, derart, dass der Rechner C programmgesteuert abhängig von dem jeweils aktuellen Messergebnis eine automatische z-Justierung der Liege durchführen kann.

Es wurde vorstehend davon gesprochen, dass im Rahmen der Abstandsmessung der Abstand zwischen einem gegebenen Augenpunkt und einem festgelegten Referenzpunkt im Koordinatensystem der laserchirurgischen Vorrichtung gemessen wird. Dies ist gleichbedeutend mit einer Messung der z-Position des gegebenen Augenpunkts in dem Koordinatensystem des Lasersystems, da jede Angabe einer z-Position stets einen Bezugspunkt, nämlich den Ursprung des Koordinatensystems, benötigt. Insoweit kann vereinfacht davon gesprochen werden, dass die Messeinrichtung 34 die z-Position des gegebenen Augenpunkts, beispielsweise des Hornhaut-Apex, misst und der Rechner C die gemessene z-Position dieses Augenpunkts mit der z-Position des Strahlfokus vergleicht. Der Hinweis auf den Referenzpunkt bei der Abstandsmessung ist insoweit nichts weiter als eine Klarstellung, dass auch jede Angabe einer absoluten Koordinatenposition stets als Abstand von einem Referenzpunkt des Koordinatensystems aufzufassen ist.

## Patentansprüche

1. Vorrichtung für die Laserablation einer humanen Kornea, mit
- einer im UV-Wellenlängenbereich strahlenden Excimer-Laserstrahlquelle (20) zur Abgabe eines fokussierten Behandlungslaserstrahls (20'),
- einer kohärenzoptischen interferometrischen Messeinrichtung (34) zum Messen des Abstands eines vorgegebenen Messpunkts oder Messbereichs eines zu behandelnden Auges (10) von einem in bekanntem Zusammenhang zur Fokuslage des Behandlungslaserstrahls stehenden Referenzpunkt (44), und
- einer Auswerte- und Steuereinheit (C), welche dazu eingerichtet ist, auf Grundlage der Abstandsmessdaten der Messeinrichtung das Zusammenfallen oder Auseinanderfallen eines gewünschten Einwirkorts des Behandlungslaserstrahls am oder im Auge und der Fokuslage des Behandlungslaserstrahls zu erfassen und im Fall des Auseinanderfallens eine Anzeige eines Justierbedarfs auf einer Anzeigeeinheit (50) zu bewirken, wobei der gewünschte Einwirkort in bekannter relativer z-Position zu dem vorgegebenen Messpunkt bzw. Messbereich liegt, wobei z die Ausbreitungsrichtung des Behandlungslaserstrahls bezeichnet, wobei der angezeigte Justierbedarf Richtung und Maß der nötigen vertikalen Verstellung des Patienten angibt.

2. Vorrichtung für die Laserablation einer humanen Kornea, mit
- einer im UV-Wellenlängenbereich strahlenden Excimer-Laserstrahlquelle (20) zur Abgabe eines fokussierten Behandlungslaserstrahls (20'),
- einer kohärenzoptischen interferometrischen Messeinrichtung (34) zum Messen des Abstands eines vorgegebenen Messpunkts oder Messbereichs eines zu behandelnden Auges (10) von einem in bekanntem Zusammenhang zur Fokuslage des Behandlungslaserstrahls stehenden Referenzpunkt (44),
- einem Verstellmechanismus für eine Patientenliege und
- einer mit dem Verstellmechanismus gekoppelten Auswerte- und Steuereinheit (C), welche dazu eingerichtet ist, auf Grundlage der Abstandsmessdaten der Messeinrichtung das Zusammenfallen oder Auseinanderfallen eines gewünschten Einwirkorts des Behandlungslaserstrahls am oder im Auge und der Fokuslage des Behandlungslaserstrahls zu erfassen und im Fall des Auseinanderfallens programmgesteuert eine automatische vertikale Justierung der Patientenliege zu bewirken, wobei der gewünschte Einwirkort in bekannter relativer z-Position zu dem vorgegebenen Messpunkt bzw. Messbereich liegt, wobei z die Ausbreitungsrichtung des Behandlungslaserstrahls bezeichnet,
wobei die Auswerte- und Steuereinheit (C) dazu eingerichtet ist, die Abgabe des Behandlungslaserstrahls (20') abhängig davon zu gestatten, dass der gewünschte Einwirkort und die Fokuslage im wesentlichen zusammenfallen.

3. Vorrichtung nach Anspruch 1 oder 2,
**dadurch gekennzeichnet, dass** die Messeinrichtung (34) dazu eingerichtet ist, den Abstand eines Punkts auf oder unter der Hornhautoberfläche des zu behandelnden Auges (10) von dem Referenzpunkt (44) zu messen.

4. Vorrichtung nach einem der vorhergehenden Ansprüche,
**dadurch gekennzeichnet, dass** die Auswerte- und Steuereinheit (C) dazu eingerichtet ist, die Ergebnisse während der Laserbehandlung des Auges durchgeführter Abstandsmessungen zu speichern oder/und auf einer Anzeigeeinheit (50) anzuzeigen.

5. Vorrichtung nach Anspruch 1,
**dadurch gekennzeichnet, dass** die Auswerte- und Steuereinheit (C) dazu eingerichtet ist, die Abgabe des Behandlungslaserstrahls (20') abhängig davon zu gestatten, dass der gewünschte Einwirkort und die Fokuslage im wesentlichen zusammenfallen.

6. Vorrichtung nach einem der vorhergehenden Ansprüche,
**dadurch gekennzeichnet, dass** die Auswerte- und Steuereinheit (C) dazu eingerichtet ist, die Abgabe des Behandlungslaserstrahls (20') abhängig davon zu unterbrechen, dass der gewünschte Einwirkort und die Fokuslage auseinanderfallen.

7. Vorrichtung nach einem der vorhergehenden Ansprüche,
**dadurch gekennzeichnet, dass** die Messeinrichtung (34) den Abstand mit optischer Kurzkohärenz-Reflektometrie misst.

## Claims

1. Apparatus for the laser ablation of a human cornea, comprising
- an excimer laser beam source (20) that radiates in the UV wavelength range, for emitting a focussed treatment laser beam (20'),
- a coherence-optical interferometric measuring device (34) for measuring the distance of a given measurement point or measurement region of an eye (10) to be treated from a reference point (44) that has a known relationship with the focal position of the treatment laser beam, and
- an evaluation and control unit (C), which is configured to capture, on the basis of the distance measurement data of the measuring device, the coincidence or non-coincidence of a desired action location of the treatment laser beam at or in the eye and the focal position of the treatment laser beam and, in the case of non-coincidence, cause an indication of an adjustment requirement on an indicating unit (50), wherein the desired action location lies at a known relative z-position in relation to the predetermined measurement point or measurement region, where z denotes the propagation direction of the treatment laser beam, wherein the indicated adjustment requirement specifies direction and dimension of the required vertical adjustment of the patient.

2. Apparatus for the laser ablation of a human cornea, comprising
- an excimer laser beam source (20) that radiates in the UV wavelength range, for emitting a focussed treatment laser beam (20'),
- a coherence optical interferometric measuring device (34) for measuring the distance of a given measurement point or measurement region of an eye (10) to be treated from a reference point (44) that has a known relationship with the focal position of the treatment laser beam,
- an adjustment mechanism for a patient couch, and
- an evaluation and control unit (C) that is coupled to the adjustment mechanism and configured to capture, on the basis of the distance measurement data of the measuring device, the coincidence or non-coincidence of a desired action location of the treatment laser beam at or in the eye and the focal position of the treatment laser beam and, in the case of non-coincidence, cause an automatic vertical adjustment of the patient couch by program control, wherein the desired action location lies at a known relative z-position in relation to the predetermined measurement point or measurement region, where z denotes the propagation direction of the treatment laser beam, wherein the evaluation and control unit (C) is configured to allow the emission of the treatment laser beam (20') provided that the desired action location and the focal position substantially coincide.

3. Apparatus according to Claim 1 or 2,
**characterized in that** the measuring device (34) is configured to measure the distance of a point at or under the corneal surface of the eye (10) to be treated from the reference point (44).

4. Apparatus according to any one of the preceding claims,
**characterized in that** the evaluation and control unit (C) is configured to store the results of distance measurements performed during the laser treatment of the eye or/and indicate these on an indicating unit (50).

5. Apparatus according to Claim 1,
**characterized in that** the evaluation and control unit (C) is configured to allow the emission of the treatment laser beam (20') provided that the desired action location and the focal position substantially coincide.

6. Apparatus according to any one of the preceding claims,
**characterized in that** the evaluation and control unit (C) is configured to interrupt the emission of the treatment laser beam (20') provided that the desired action location and the focal position are non-coincident.

7. Apparatus according to any one of the preceding claims,
**characterized in that** the measuring device (34) measures the distance using optical low-coherence reflectometry.

## Revendications

1. Dispositif destiné à l'ablation au laser d'une cornée humaine, avec :
- une source de faisceau laser (20) à excimère, laquelle transmet le faisceau laser dans la plage de longueur d'onde des UV et laquelle est destinée à la production d'un faisceau laser focalisé de traitement (20') ;
- un mécanisme de mesure (34) d'interférométrie en cohérence optique, lequel est destiné à la mesure de la distance d'un point de mesure ou d'une plage de mesure prédéfini (e) d'un oeil (10) à traiter par un point de référence (44) qui se trouve dans un contexte connu, en vue de la position de focalisation du faisceau laser de traitement ; et
- une unité de commande et d'évaluation (C), laquelle est configurée en vue de détecter, sur la base des données de mesure de la distance fournies par le mécanisme de mesure, l'affaissement ou la dislocation d'une zone d'application souhaitée du faisceau laser de traitement au niveau de l'oeil ou dans l'oeil, ainsi que la position de focalisation du faisceau laser de traitement et, dans le cas d'une dislocation, en vue de donner lieu à un affichage d'un besoin de réglage sur une unité d'affichage (50) ;
dans lequel la zone d'application souhaitée se situe dans une position « z » relative connue par rapport au point de mesure prédéfini, respectivement par rapport à la plage de mesure prédéfinie ;
dans lequel « z » désigne la direction de propagation du faisceau laser de traitement ;
dans lequel le besoin de réglage affiché indique la direction et l'ampleur de l'ajustement vertical nécessaire du patient.

2. Dispositif destiné à l'ablation au laser d'une cornée humaine, avec :
- une source de faisceau laser (20) à excimère, laquelle transmet le faisceau laser dans la plage de longueur d'onde des UV et laquelle est destinée à la production d'un faisceau laser focalisé de traitement (20') ;
- un mécanisme de mesure (34) d'interférométrie en cohérence optique, lequel est destiné à la mesure de la distance d'un point de mesure ou d'une plage de mesure prédéfini (e) d'un oeil (10) à traiter par un point de référence (44) qui se trouve dans un contexte connu, en vue de la position de focalisation du faisceau laser de traitement ;
- un mécanisme de réglage pour une table de salle d'opération ; et
- une unité de commande et d'évaluation (C), laquelle est couplée au mécanisme de réglage et laquelle est configurée en vue de détecter, sur la base des données de mesure de la distance fournies par le mécanisme de mesure, l'affaissement ou la dislocation d'une zone d'application souhaitée du faisceau laser de traitement au niveau de l'oeil ou dans l'oeil, ainsi que la position de focalisation du faisceau laser de traitement et, dans le cas d'une dislocation, en vue de donner lieu à un ajustement vertical automatique de la table de salle d'opération, lequel ajustement est piloté par un programme ;
dans lequel la zone d'application souhaitée se situe dans une position « z » relative connue par rapport au point de mesure prédéfini, respectivement à la plage de mesure prédéfinie ;
dans lequel « z » désigne la direction de propagation du faisceau laser de traitement ;
dans lequel l'unité de commande et d'évaluation (C) est configurée en vue de permettre la production du faisceau laser de traitement (20') en fonction, pour l'essentiel, d'un affaissement de la zone d'application souhaitée et de la position de focalisation.

3. Dispositif selon la revendication 1 ou 2,
**caractérisé en ce que** le mécanisme de mesure (34) est configuré en vue de mesurer la distance d'un point qui se trouve sur ou au-dessous de la surface de la cornée de l'oeil (10) à traiter par rapport au point de référence (44).

4. Dispositif selon l'une des revendications précédentes,
**caractérisé en ce que** l'unité de commande et d'évaluation (C) est configurée en vue d'enregistrer les résultats des mesures de la distance qui sont réalisées pendant le traitement de l'oeil au laser ou/et en vue d'afficher ces résultats une unité d'affichage (50).

5. Dispositif selon la revendication 1,
**caractérisé en ce que** l'unité de commande et d'évaluation (C) est configurée en vue de permettre la production du faisceau laser de traitement (20') en fonction, pour l'essentiel, d'un affaissement de la zone d'application souhaitée et de la position de focalisation.

6. Dispositif selon l'une des revendications précédentes,
**caractérisé en ce que** l'unité de commande et d'évaluation (C) est configurée en vue d'interrompre la production du faisceau laser de traitement (20') en fonction d'une dislocation de la zone d'application souhaitée et de la position de focalisation.

7. Dispositif selon l'une des revendications précédentes,
**caractérisé en ce que** le mécanisme de mesure (34) mesure la distance avec une réflectométrie optique à faible cohérence.
